(19) Europäisches Patentamt
European Patent Office
Office européen des brevets

(11) Veröffentlichungsnummer: **0 066 761**
**A1**

(12) **EUROPÄISCHE PATENTANMELDUNG**

(21) Anmeldenummer: **82104486.4**

(22) Anmeldetag: **22.05.82**

(51) Int. Cl.³: **C 07 C 33/50**, C 07 C 33/48, C 07 C 33/42, C 07 C 33/44, C 07 C 35/52, C 07 C 43/23, A 01 N 31/04 // C07C29/62

(30) Priorität: **04.06.81 DE 3122176**

(43) Veröffentlichungstag der Anmeldung: **15.12.82 Patentblatt 82/50**

(84) Benannte Vertragsstaaten: **BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Jäger, Gerhard, Dr., Gellertstrasse 18, D-5090 Leverkusen 1 (DE)**
Erfinder: **Brandes, Wilhelm, Dr., Eichendorffstrasse 3, D-5653 Leichlingen (DE)**
Erfinder: **Frohberger, Paul-Ernst, Dr., Willi-Baumeister-Strasse 5, D-5090 Leverkusen 1 (DE)**

(54) **Substituierte 1-Jod-1-butin-4-ole, Verfahren zu ihrer Herstellung und ihre Verwendung als Pflanzenschutzmittel.**

(57) Die Erfindung betrifft neue substituierte 1-Jod-1-butin-4-ole der allgemeinen Formel,

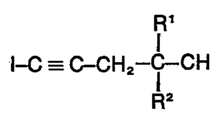

in welcher

R¹ für Alkyl, gegebenenfalls substituiertes Aryl, Aralkyl oder Aryloxyalkyl steht,

R² für Alkyl mit mehr als einem C-Atom, Halogenalkyl, gegebenenfalls substituiertes Cycloalkyl oder Aryl steht und für den Fall, daß R¹ für gegebenenfalls substituiertes Aryl, Aralkyl oder Aryloxyalkyl steht, außerdem für Methyl steht oder

R¹ und R² gemeinsam mit dem C-Atom, an das sie gebunden sind, für Fluoren-9-yl stehen,

ein Verfahren zu ihrer Herstellung sowie ihre Verwendung als Pflanzenschutzmittel.

Die neuen 1-Jod-1-butin-4-ole werden erhalten, wenn man geeignete 1-Butin-4-ole in Gegenwart einer basischen Verbindung mit Jod umsetzt.

EP 0 066 761 A1

BAYER AKTIENGESELLSCHAFT
Zentralbereich
Patente, Marken und Lizenzen

5090 Leverkusen, Bayerwerk
Eck/bc/c

# Substituierte 1-Jod-1-butin-4-ole, Verfahren zu ihrer Herstellung und ihre Verwendung als Pflanzenschutzmittel

Die vorliegende Erfindung betrifft neue substituierte 1-Jod-1-butin-4-ole, ein Verfahren zu ihrer Herstellung, sowie ihre Verwendung als Pflanzenschutzmittel.

Es ist bekannt, daß bestimmte Jodpropargylcarbinolverbindungen, beispielsweise 4-(4-Chlorphenyl)-1-jod-1-butin-4-ol und 1-Jod-4-phenyl-1-butin-4-ol (Deutsche Auslegeschrift 1 217 368) fungizide Eigenschaften aufweisen. Die Wirkung dieser Verbindungen ist jedoch, insbesondere bei niedrigen Aufwandmengen und -konzentrationen nicht immer ganz befriedigend.

Es wurden neue substituierte 1-Jod-1-butin-4-ole der allgemeinen Formel (I),

$$I-C\equiv C-CH_2-\overset{R^1}{\underset{R^2}{C}}-OH \qquad (I)$$

in welcher

$R^1$ für Alkyl, gegebenenfalls substituiertes Aryl, Aralkyl oder Aryloxyalkyl steht,

$R^2$ für Alkyl mit mehr als einem C-Atom, Halogenalkyl, gegebenenfalls substituiertes Cycloalkyl oder Aryl steht und für den Fall, daß $R^1$ für gegebenenfalls substituiertes Aryl, Aralkyl oder Aryloxyalkyl steht, außerdem für Methyl steht oder

$R^1$ und $R^2$ gemeinsam mit dem C-Atom an das sie gebunden sind für Fluoren-9-yl stehen,

gefunden.

Weiterhin wurde gefunden, daß man die neuen substituierten 1-Jod-1-butin-4-ole der Formel (I)

$$I-C\equiv C-CH_2-\underset{R^2}{\overset{R^1}{C}}-OH \qquad (I)$$

in welcher

$R^1$ für Alkyl, gegebenenfalls substituiertes Aryl, Aralkyl oder Aryloxyalkyl steht;

$R^2$ für Alkyl mit mehr als einem C-Atom, Halogenalkyl, gegebenenfalls substituiertes Cycloalkyl oder Aryl steht und für den Fall, daß $R^1$ für gegebenenfalls substituiertes Aryl, Aralkyl oder Aryloxyalkyl steht, außerdem für Methyl steht oder

$R^1$ und $R^2$ gemeinsam mit dem C-Atom an das sie gebunden sind für Fluoren-9-yl stehen,

erhält, wenn man 1-Butin-4-ole der Formel (II),

$$H-C\equiv C-CH_2-\overset{R^1}{\underset{R^2}{C}}-OH \qquad (II)$$

in welcher
$R^1$ und $R^2$ die oben angegebenen Bedeutungen haben,

mit Jod in Gegenwart einer basischen Verbindung und gegebenenfalls in Gegenwart eines Verdünnungsmittels umsetzt.

Die erfindungsgemäßen Verbindungen zeigen überraschenderweise eine höhere fungizide Wirkung als die bekannten Wirkstoffe 4-(4-Chlorphenyl)-1-jod-1-butin-4-ol und 1-Jod-4-phenyl-1-butin-4-ol. Die erfindungsgemäßen Wirkstoffe stellen somit eine Bereicherung der Technik dar.

Die erfindungsgemäßen substituierten 1-Jod-1-butin-4-ole sind durch die Formel (I) allgemein definiert. Bevorzugt sind diejenigen Verbindungen der Formel (I), in denen

$R^1$ für gegebenenfalls substituiertes Aryl mit 6 bis 10 C-Atomen, Aralkyl und Aryloxyalkyl mit 6 bis 10 C-Atomen im Arylteil und 1 bis 4 C-Atomen im Alkylteil steht,

$R^2$ für $C_1$-$C_{10}$-Alkyl, $C_1$-$C_{10}$-Halogenalkyl mit 1 bis 13 Halogenatomen, gegebenenfalls substituiertes Cycloalkyl mit 3 bis 7 C-Atomen, oder Aryl mit 6 bis 10 C-Atomen steht oder

$R^1$ und $R^2$ gemeinsam mit dem C-Atom an das sie gebunden sind für Fluoren-9-yl stehen.

Für den Fall, daß $R^1$ für Alkyl steht, sei bevorzugt $C_1$-$C_{10}$-Alkyl wie Methyl, Ethyl, Propyl, tert.-Butyl, Cyclohexyl, Oktyl, Decyl genannt.

Als Substituenten seien Halogen wie Fluor, Chlor, Brom und Jod, $C_1$-$C_6$-Alkyl, $C_1$-$C_4$-Halogenalkyl und Halogenalkoxy mit 1 bis 7 Halogenatomen wie Fluor und Chlor oder Aryl wie Phenyl und Naphthyl, das seinerseits gegebenenfalls die genannten Substituenten tragen kann, genannt.

Bevorzugte Substituenten sind Halogen wie Fluor, Chlor und Brom, $C_1$-$C_4$-Alkyl, $C_1$-$C_2$-Halogenalkyl und Halogenalkoxy mit 1 bis 3 Halogenatomen wie Fluor und Chlor und Phenyl, das seinerseits gegebenenfalls die genannten Substituenten tragen kann.

Bevorzugt tragen $R^1$ und unabhängig davon $R^2$ dann Substituenten, wenn sie für Aryl, Aralkyl und Aryloxyalkyl stehen. Ganz besonders bevorzugt sind diejenigen 1-Jod-1-butin-4-ole der Formel (I), in welcher

$R^1$ für gegebenenfalls substituiertes Phenyl, Benzyl oder Phenyloxymethyl steht,

$R^2$ für $C_1$-$C_4$-Alkyl wie Methyl, Ethyl, Propyl, iso-Propyl, Butyl, sek.-Butyl, tert.-Butyl, $C_1$-$C_4$-Halogenalkyl mit 1 bis 3 Halogenatomen, Cyclohexyl oder gegebenenfalls substituiertes Phenyl steht oder

$R^1$ und $R^2$ gemeinsam mit dem C-Atom an das sie gebunden sind für Fluoren-9-yl stehen.

Als Substituenten kommen die oben genannten in Frage.

Als Beispiele für die erfindungsgemäßen 1-Jod-1-butin-4-ole der Formel (I) seien die folgenden Verbindungen der Tabelle 1 neben den Herstellungsbeispielen genannt:

Tabelle 1

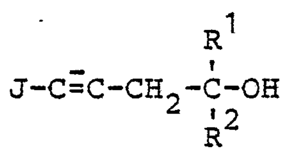

(I)

| $R^1$ | $R^2$ |
|---|---|
| 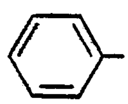 | 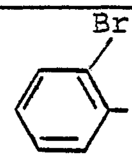 |
| 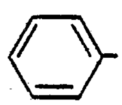 | 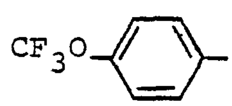 |
| 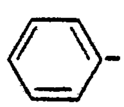 | 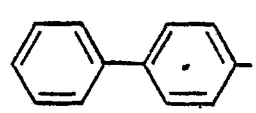 |
| 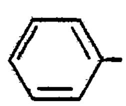 | 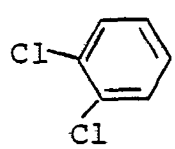 |

| $R^1$ | $R^2$ |
|---|---|
| 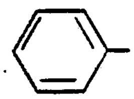 | $n-C_3H_7-$ |
| 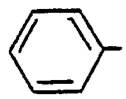 | $n-C_4H_9-$ |
| 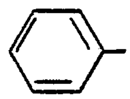 | $t-C_4H_9$ |
| 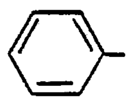 | $i-C_4H_9$ |
| 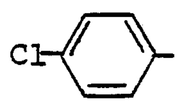 | 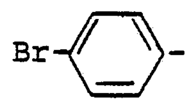 |
| 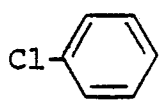 | 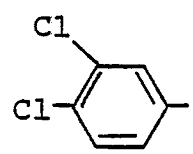 |
| 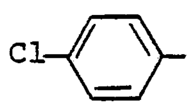 | 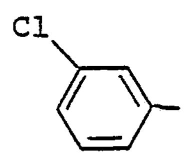 |
| 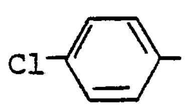 | $n-C_3H_7$ |
| 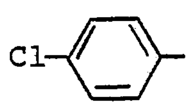 | $n-C_4H_9$ |

| $R^1$ | $R^2$ |
|---|---|
| 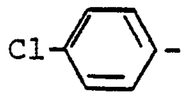 | 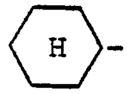 |
| 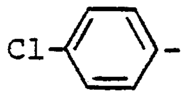 | $i-C_4H_9$ |
| 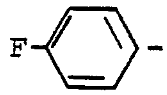 | 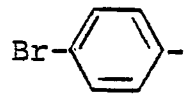 |
| 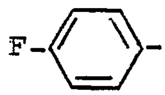 | 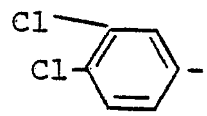 |
| 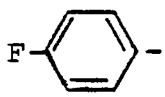 | 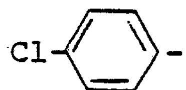 |
| 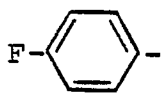 | 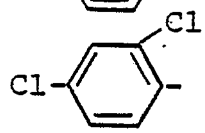 |
| 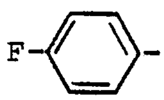 | $n-C_3H_7$ |
| 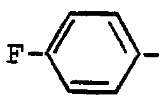 | $n-C_4H_9$ |
| 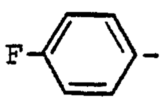 | $i-C_4H_9$ |
| 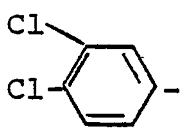 | 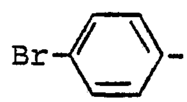 |
| 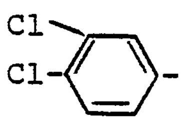 | 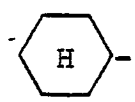 |
| 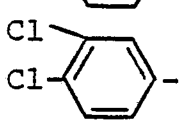 | $CH_3-$ |
| 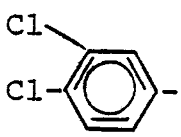 | $n-C_3H_7$ |

| $R^1$ | $R^2$ |
|---|---|
| 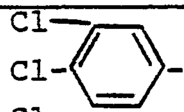 | i-$C_3H_7$ |
| 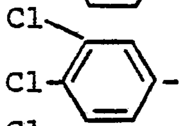 | n-$C_4H_9$ |
| 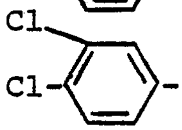 | i-$C_4H_9$ |
| 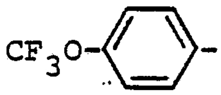 | 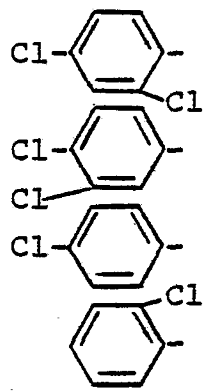 |
| 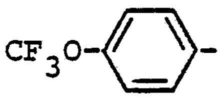 | |
| 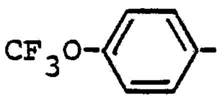 | |
| 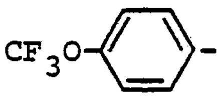 | |
| 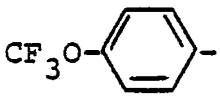 | 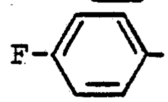 |
| 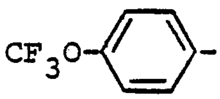 | $C_2H_5$ |
| 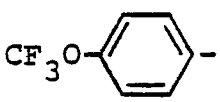 | i-$C_3H_7$ |
| 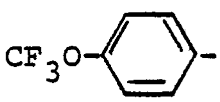 | n-$C_3H_7$ |
| 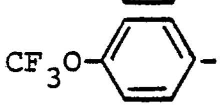 | 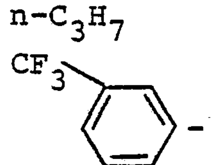 |
| 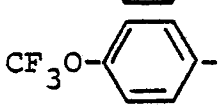 | 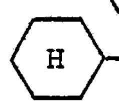 |
| 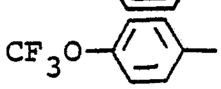 | n-$C_4H_9$ |
| 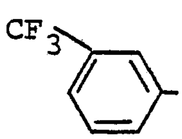 | 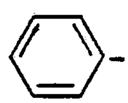 |
| 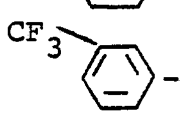 | 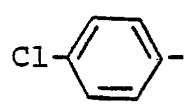 |

Durch diese Tabelle sollen mögliche Kombinationen von Resten, die unter die Definition der allgemeinen Formel (I) fallen, nicht ausgeschlossen werden.

Verwendet man beispielsweise 4-(4-Chlorphenyloxymethyl)-5,5-dimethyl-1-hexin-4-ol und Jod als Ausgangsstoffe, so kann der Reaktionsablauf durch das folgende Formelschema wiedergegeben werden:

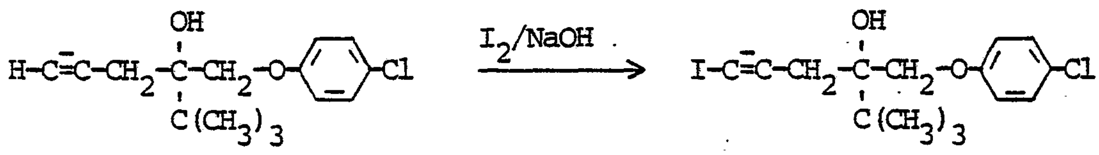


Die bei der Durchführung des erfindungsgemäßen Verfahrens als Ausgangsstoffe zu verwendenden 1-Butin-4-ole sind durch die Formel (II) allgemein definiert. In dieser Formel stehen vorzugsweise

$R^1$ für gegebenenfalls substituiertes Aryl mit 6 bis 10 C-Atomen, Aralkyl und Aryloxyalkyl mit 6 bis 10 C-Atomen im Arylteil und 1 bis 4 C-Atomen im Alkylteil;

$R^2$ für $C_1$-$C_{10}$-Alkyl, $C_1$-$C_4$-Halogenalkyl mit 1 bis 13 Halogenatomen, gegebenenfalls substituiertes Cycloalkyl mit 3 bis 7 C-Atomen, oder Aryl mit 6 bis 10 C-Atomen oder

$R^1$ und $R^2$ gemeinsam mit dem C-Atom an das sie gebunden sind für Fluoren-9-yl.

Als Substituenten seien Halogen wie Fluor, Chlor, Brom und Jod, $C_1$-$C_6$-Alkyl, $C_1$-$C_4$-Halogenalkyl und Halogenalkoxy mit 1 bis 7 Halogenatomen wie Fluor und Chlor oder Aryl wie Phenyl und Naphthyl, das seinerseits gegebenenfalls den genannten Substituenten tragen kann, genannt.

Bevorzugte Substituenten sind Halogen wie Fluor, Chlor und Brom, $C_1$-$C_4$-Alkyl, $C_1$-$C_2$-Halogenalkyl und Halogenalkoxy mit 1 bis 3 Halogenatomen wie Fluor und Chlor und Phenyl, das seinerseits gegebenenfalls die genannten Substituenten tragen kann.

Bevorzugt tragen $R^1$ und unabhängig davon $R^2$ dann Substituenten, wenn sie für Aryl, Aralkyl und Aryloxyalkyl stehen. Ganz besonders bevorzugt sind diejenigen 1-Butin-4-ole der Formel (II), in welcher

$R^1$ für gegebenenfalls substituiertes Phenyl-, Benzyl- oder Phenyloxymethyl steht,

$R^2$ für $C_1$-$C_4$-Alkyl wie Methyl, Ethyl, Propyl, iso-Propyl, Butyl, sek.-Butyl, tert.-Butyl, $C_1$-$C_4$-Halogenalkyl mit 1 bis 3 Halogenatomen, Cyclohexyl oder gegebenenfalls substituiertes Phenyl steht oder

$R^1$ und $R^2$ gemeinsam mit dem C-Atom an das sie gebunden sind, für Fluoren-9-yl stehen.

Als Substituenten kommen die oben genannten in Frage.

Die 1-Butin-4-ole der Formel (II) sind bekannt, oder können auf bekannte Art und Weise hergestellt werden

(Deutsche Auslegeschrift 1 217 368; J.Agr. Food Chem. 18, 78 (1970) und Liebigs Ann.Chem. 682, 62 (1965)).

Man erhält sie beispielsweise, indem man die entsprechenden Ketone in Gegenwart von aktiviertem Aluminium in einem aprotischen organischen Lösungsmittel, beispielsweise in Tetrahydrofuran mit Propargylhalogeniden bei Temperaturen zwischen -70 und 30°C umsetzt.

Für die erfindungsgemäße Umsetzung kommen als Verdünnungsmittel vorzugsweise protische Lösungsmittel in Frage. Hierzu gehören vorzugsweise Alkohole, wie Methanol, Ethanol, Propanol, Ispropanol und Glykolmonomethylether. Gegebenenfalls kann auch in Mischungen derselben mit anderen Verdünnungsmitteln, beispielsweise Wasser oder mit organischen Aminen, wie Pyridin, Chinolin oder Picolinen, gearbeitet werden.

Die erfindungsgemäße Umsetzung wird in Gegenwart von basischen Verbindungen ausgeführt. Als basische Verbindungen können Metallhydroxide, beispielsweise Hydroxide von Alkali-, Erdalkali-, und Metallen der III. Hauptgruppe des Periodensystems nach Mendelejew eingesetzt werden. Bevorzugt werden Alkali- und Erdalkalihydroxide wie Natrium-, Kalium-, Calcium- und Bariumhydroxid eingesetzt. Besonders bevorzugt werden Natrium- und Kaliumhydroxid eingesetzt. Die basischen Verbindungen werden bevorzugt in einem Verdünnungsmittel gelöst eingesetzt. Besonders bevorzugt sind wäßrige Lösungen.

Die Reaktionstemperaturen können beim erfindungsgemäßen Verfahren in einem größeren Bereich variiert werden. Im allgemeinen arbeitet man von 0 bis 50°C, vorzugsweise von 20 bis 30°C.

Bei der Durchführung des erfindungsgemäßen Verfahrens können Jod und eine basische Verbindung simultan zum vorgelegten 1-Butin-4-ol der Formel (II) gegeben werden.

Es kann auch eine Mischung von Jod mit Alkalihydroxid zum vorgelegten 1-Butin-4-ol der Formel (II) gegeben werden.

Weiterhin ist es möglich, Jod und eine basische Verbindung vorzulegen und 1-Butin-4-ol der Formel (II) zuzugeben.

In einer weiteren Variante kann 1-Butin-4-ol der Formel (II) und eine basische Verbindung vorgelegt werden. Dazu wird Jod gegeben.

Weiterhin kann es vorteilhaft sein, Jod und 1-Butin-4-ol der Formel(II) vorzulegen und dazu eine basische Verbindung zu geben.

Das erfindungsgemäße Verfahren kann kontinuierlich und diskontinuierlich ausgeführt werden.

Im allgemeinen wird das erfindungsgemäße Verfahren wie folgt ausgeführt: Zum vorgelegten 1-Butin-4-ol der

Formel (II) wird eine basische Verbindung gegeben und gleichzeitig portionsweise Jod eingetragen. Nachdem die Umsetzung beendet ist, wird auf übliche Art und Weise aufgearbeitet und das erhaltene 1-Jod-1-butin-4-ol der Formel (I) beispielsweise durch Kristallisation und/oder Destillation gereinigt.

Die im erfindungsgemäßen Verfahren eingesetzten 1-Butin-4-ole der Formel (II) können ein asymmetrisches C-Atom enthalten. In besonderen Fällen kann die Konfiguration dieses C-Atoms im Verlauf der erfindungsgemäßen Umsetzung beeinflußt werden.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Die erfindungsgemäßen Wirkstoffe haben ein breites Wirkungsspektrum. Sie können gegen pflanzenpathogene Schädlinge verwendet werden. Insbesondere können sie als Fungizide verwendet werden, beispielsweise gegen parasitäre Pilze, die oberirdische Pflanzenteile befallen oder die Pflanzen vom Boden her angreifen, sowie gegen samenübertragbare Krankheitserreger. Eine besonders gute Wirksamkeit entfalten sie gegen parasitäre Pilze auf oberirdischen Pflanzenteilen.

Als Pflanzenschutzmittel können die erfindungsgemäßen Wirkstoffe mit besonders gutem Erfolg zur Bekämpfung von Phytophthora infestans (Braunfäule der Tomate) sowie Erysiphe-Arten, wie gegen den Erreger des Getreidemehltaus (Erysiphe graminis) und zur Bekämpfung von Puccinia-Arten, wie Puccinia recondita (Getreiderost) eingesetzt werden. Gute Wirkungen werden ebenfalls erzielt bei der Bekämpfung von Reiskrankheiten, beispielsweise Pyricularia oryzae und bei der Bekämpfung schädlicher Milben.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.a., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten wie Xylol, Toluol, oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe wie Cyclohexan oder Paraffine, z.B. Erdölfraktionen, Alkohole wie Butanol oder Glykol sowie deren Ether und Ester, Ketone wie Aceton, Methylethylketon, Methylisobutylketon oder Cyclohexanon, stark polare Lösungsmittel wie Dimethylformamid und Dimethylsulfoxid sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase wie Halogenkohlenwasserstoffe sowie Butan, Propan, Stickstoff und Kohlendioxid; als feste Trägerstoffe kommen in Frage: z.B. natürliche Gesteinsmehle wie Kaoline, Tonerden, Talkum, Kreide, Quarz, Attapulgit, Montmorillonit oder Diatomeenerde und synthetische Gesteinsmehle wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische

Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nicht-ionogene und anionische Emulgatoren wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 Gew.-%.

Die erfindungsgemäßen Wirkstoffe können in den Formulierungen oder in den verschiedenen Anwendungsformen in Mischung mit anderen bekannten Wirkstoffen vorliegen wie Fungiziden, Bakteriziden, Insektiziden,

Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele

Beispiel 1

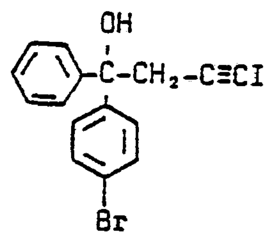

Unter gleichzeitiger portionsweiser Zugabe von 15,3 g (0,06 Mol) Jod werden bei 20 bis 25°C im Verlaufe von 20 Minuten 24 ml konzentrierte Natronlauge in eine Lösung von 15,1 g (0,05 Mol) 4-(4-Bromphenyl)-4-phenyl-1-butin-4-ol in 150 ml Methanol eingerührt. Nach 8-stündigem Stehen des Reaktionsgemisches werden die ausgeschiedenen Kristalle abgesaugt, mit Wasser und Methanol gut gewaschen und getrocknet. Man erhält 19,4 g (90,9 % der Theorie) 4-(4-Bromphenyl)-1-jod-4-phenyl-1-butin-4-ol als farblose Kristalle vom Schmelzpunkt 139 bis 140°C.

Beispiel 2

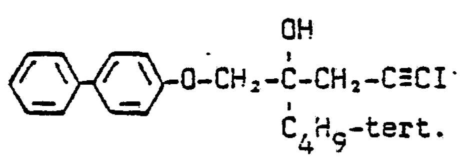

In eine Lösung von 30,8 g (0,1 Mol) 4-Biphenyloxymethyl-5,5-dimethyl-1-hexin-4-ol in 250 ml Methanol werden im Laufe von 30 Minuten bei 20°C 48 ml konzentrierte Natronlauge eingerührt und gleichzeitig portionsweise 30,5 g (0,12 Mol) Jod eingetragen. Nach

8-stündigem Rühren wird der ausgeschiedene Festkörper abfiltriert, mit wenig kaltem Methanol gewaschen und getrocknet. Man erhält so 39 g (89,8 % der Theorie) 4-Biphenyloxymethyl-5,5-dimethyl-1-Jod-1-hexin-4-ol in Form farbloser Kristalle vom Schmelzpunkt 134 bis 135°C.

In analoger Weise werden die 1-Jod-1-butin-4-ole der Formel (I) der nachfolgenden Tabelle 2 hergestellt.

Tabelle 2

$$J-C\equiv C-\underset{R^2}{\overset{R^1}{C}}-OH \qquad (I)$$

| Beispiel-Nr. | $R^1$ | $R^2$ | Physikal. Konstante |
|---|---|---|---|
| 3 | 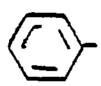 | 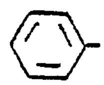 | Fp. 1o2-1o3°C |
| 4 | 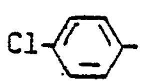 | 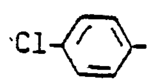 | Fp. 123-124°C |
| 5 | 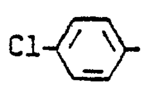 | 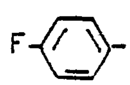 | Fp. 82 - 83°C |
| 6 | 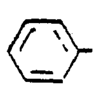 | $CH_3-$ | $n_D^{2o}$: 1,6o17 |
| 7 | 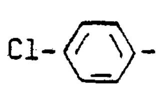 | $CH_3-$ | $n_D^{2o}$: 1,6o78 |

| Bei-spiel Nr. | $R^1$ | $R^2$ | Physikal. Konstante |
|---|---|---|---|
| 8 | 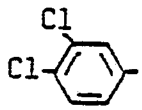 | $CH_3$- | $n_D^{2o}$: 1,61o7 |
| 9 | 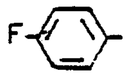 | $CH_3$- | $n_D^{2o}$: 1,5781 |
| 10 | 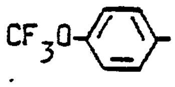 | $CH_3$- | $n_D^{2o}$: 1,5146 |
| 11 | 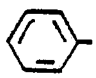 | 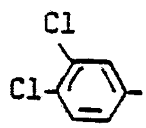 | zähes Öl |
| 12 | 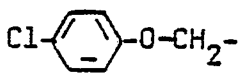 | $C_4H_9$-tert. | $n_D^{2o}$: 1,57o8 |
| 13 | 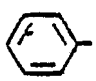 | 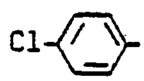 | Fp. 1o7°C |
| 14 | 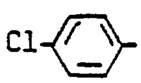 | 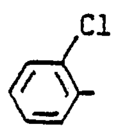 | zähes Öl |
| 15 | 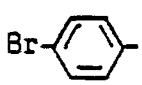 | $CH_3$- | $n_D^{2o}$: 1,622o |
| 16 | 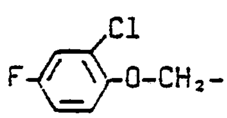 | $C_4H_9$-tert. | $n_D^{2o}$: 1,5575 |

| Beispiel Nr. | $R^1$ | $R^2$ | Physikal. Konstante |
|---|---|---|---|
| 17 | 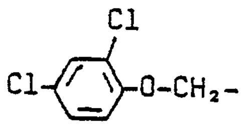 | $C_4H_9$-tert. | $n_D^{20}$: 1,5753 |
| 18 | 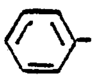 | 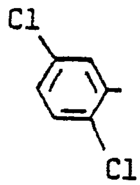 | zähes Öl |
| 19 | 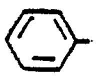 | 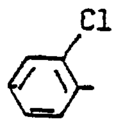 | zähes Öl |
| 20 | 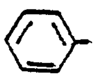 | 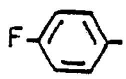 | $n_D^{20}$: 1,6174 |
| 21 | 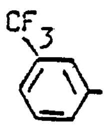 | $CH_3$- | $n_D^{20}$: 1,5336 |
| 22 | 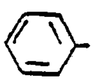 | 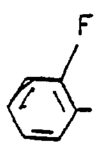 | Fp. 81-82°C |
| 23 | 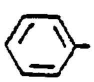 | 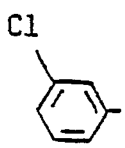 | $n_D^{20}$: 1,6280 |
| 24 | 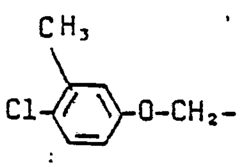 | $C_4H_9$-tert. | $n_D^{20}$: 1,5629 |
| 25 | 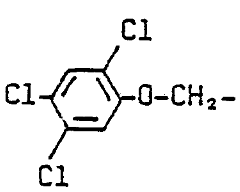 | $C_4H_9$-tert. | Fp. 110°C |

| Beispiel Nr. | $R^1$ | $R^2$ | Physikal. Konstante |
|---|---|---|---|
| 26 | 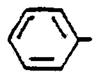 | $C_3H_7$-iso | $n_D^{20}$: 1,5839 |
| 27 | 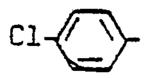 | $C_3H_7$-iso | $n_D^{20}$: 1,5878 |
| 28 | 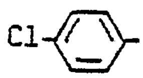 | $C_2H_5$- | $n_D^{20}$: 1,5752 |
| 29 | 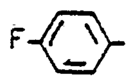 | $C_3H_7$-iso | $n_D^{20}$: 1,5668 |
| 30 | 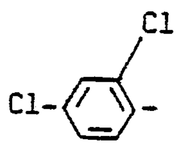 | $C_2H_5$- | $n_D^{20}$: 1,6039 |
| 31 | 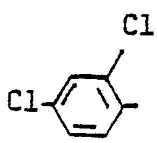 | $CH_3$ | $n_D^{20}$: 1,6148 |
| 32 | 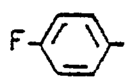 | 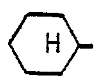 | $n_D^{20}$: 1,5771 |
| 33 | 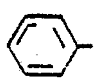 | 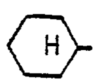 | $n_D^{20}$: 1,5912 |
| 34 | 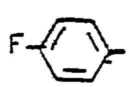 | $C_2H_5$ - | $n_D^{20}$: 1,5702 |
| 35 | 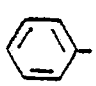 | $C_2H_5$- | $n_D^{20}$: 1,5908 |

| Beispiel Nr. | $R^1$ | $R^2$ | Physikal. Konstante |
|---|---|---|---|
| 36 | 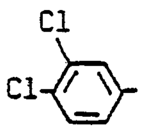 | $C_2H_5$- | $n_D^{20}$: 1,6060 |
| 37 | 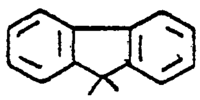 | | Fp. 133-134°C |
| 38 | 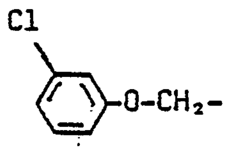 | $C_4H_9$-tert. | |
| 39 | 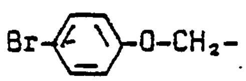 | $-C(CH_2F)_2(CH_3)$ | |
| 40 | 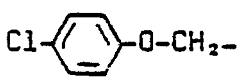 | 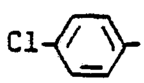 | |
| 41 | 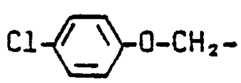 | 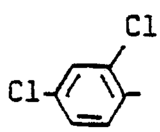 | $n_D^{20}$: 1,6279 |
| 42 | 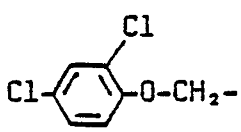 | 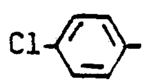 | |
| 43 | 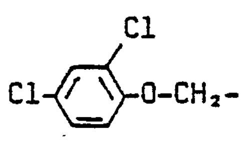 | 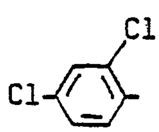 | |
| 44 | 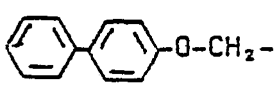 | 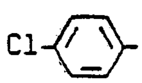 | |

| Beispiel Nr. | $R^1$ | $R^2$ | Physikal.Konstante |
|---|---|---|---|
| 45 | 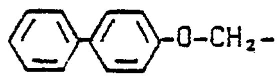 | 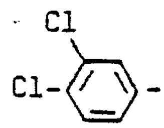 | |
| 46 | 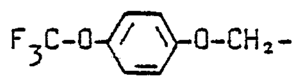 | 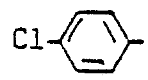 | |
| 47 | 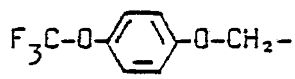 | 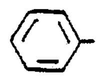 | |
| 48 | 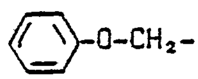 | 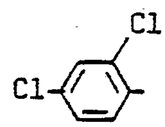 | |
| 49 | 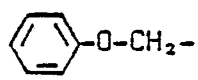 | 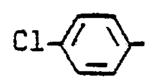 | |
| 5o | 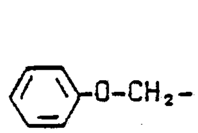 | 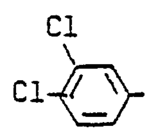 | |
| 51 | 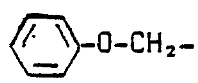 | 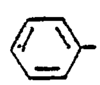 | |
| 52 | 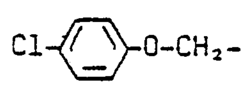 | 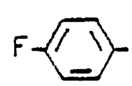 | $n_D^{20}$: 1,6055 |
| 53 | 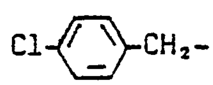 | $CF_3$ | $n_D^{20}$: 1,5468 |
| 54 | 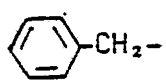 | $CF_3$ | $n_D^{20}$: 1,5421 |

Herstellung der Vorprodukte

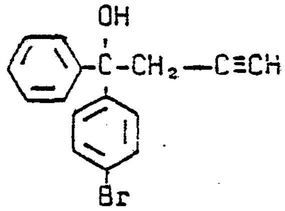

13 g Aluminium (schuppenförmig) werden mit je einer Spatelspitze Quecksilber-II-chlorid und Jod versetzt, mit 60 ml trockenem Tetrahydrofuran versetzt und 8 Stunden stehen gelassen. Anschließend wird bei 55 bis 60°C eine Lösung von 84,5 g (0,71 Mol) Propargylbromid in 85 ml absolutem Tetrahydrofuran innerhalb von 45 Minuten unter Rühren zugetropft. Man kühlt auf -60°C ab und tropft eine Lösung von 130,5 g (0,5 Mol) 4-Brombenzophenon in 120 ml trockenem Tetrahydrofuran innerhalb einer Stunde unter Rühren zu. Man erwärmt auf 0°C, läßt bei dieser Temperatur eine Stunde rühren und versetzt anschließend mit 170 ml gesättigter Ammoniumchloridlösung. Es wird danach mit Essigester extrahiert, mit Wasser gewaschen, über Natriumsulfat getrocknet, filtriert und unter Vakuum das Lösungsmittel abdestilliert. Der Rückstand wird mit wenig Petrolether angerieben. Man erhält so 119,2 g (79,2 % der Theorie) an 4-(4-Bromphenyl)-4-phenyl-1-butin-4-ol als farblose Kristalle vom Schmelzpunkt 78 bis 79°C.

Herstellung des Vorprodukts

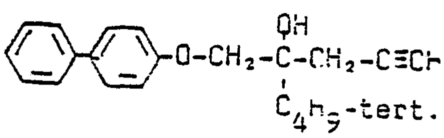

Man läßt auf 13 g Aluminium (in Schuppenform) je eine Spatelspitze Quecksilber-II-chlorid und Jod für etwa 10 Minuten einwirken. Anschließend versetzt man mit 50 ml trockenem Tetrahydrofuran und läßt das Gemisch 20 Stunden rühren. Danach wird eine Lösung von 84,5 g (0,71 Mol) Propargylbromid, gelöst in 85 ml Tetrahydrofuran, zugetropft. Die einsetzende exotherme Reaktion wird durch leichte Außenkühlung bei 55 bis 60°C gehalten. Man kühlt die Lösung auf -60°C ab und tropft im Laufe von 30 Minuten 134,2 g (0,5 Mol) 1-Biphenyloxy-3,3-dimethyl-butanon-2 in 165 ml trockenem Tetrahydrofuran zu. Nach 1-stündigem Rühren bei 0°C wird das Reaktionsgemisch in 170 ml gesättigte Ammoniumchlorid-Lösung eingerührt. Man extrahiert zweimal mit je 200 ml Essigester, wäscht die organische Phase mit Wasser und engt nach dem Trocknen der Lösung über wasserfreiem Natriumsulfat ein. Nach Filtration und anschließendes Anreiben des Rückstandes mit Petrolether erhält man 127,1 g (82,5 % der Theorie) 4-Biphenyloxymethyl-5,5-dimethylhexin-4-ol vom Schmelzpunkt 74 bis 76°C.

In analoger Weise werden die 1-Butin-4-ole der Formel (II) der nachfolgenden Tabelle hergestellt.

Tabelle 3

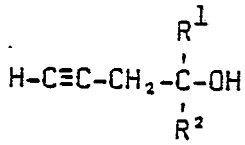 (II)

| $R^1$ | $R^2$ | Physikal.Konstante |
|---|---|---|
| 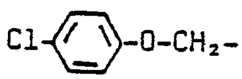 | $C_4H_9$-tert. | $n_D^{20}$: 1,5285 |
| 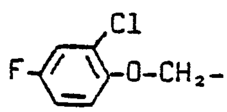 | $C_4H_9$-tert. | $n_D^{20}$: 1,5163 |
| 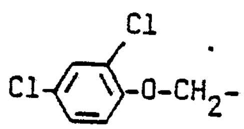 | $C_4H_9$-tert | $n_D^{20}$: 1,5391 |
| 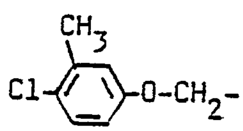 | $C_4H_9$-tert | $n_D^{20}$: 1,5294 |
| 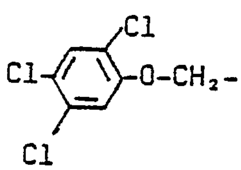 | $C_4H_9$-tert. | Fp. 71°C |
| 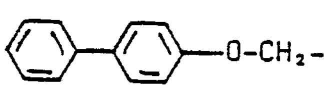 | $C_4H_9$-tert | Fp.73-76°C |

| $R^1$ | $R^2$ | Physikal.Konstante |
|---|---|---|
| 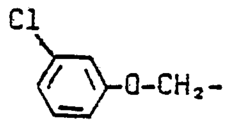 | $C_4H_9$-tert | $n_D^{20}$: 1,5293 |
| 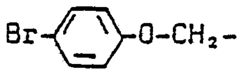 | $-C(CH_2F)_2(CH_3)$ | $n_D^{20}$: 1,538o |
| 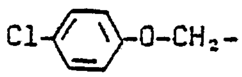 | 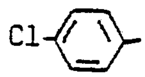 | |
| 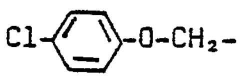 | 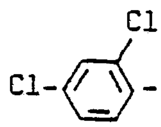 | $n_D^{20}$: 1,5965 |
| 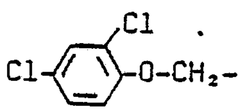 | 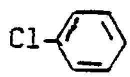 | |
| 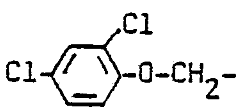 | 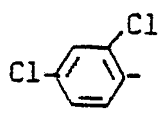 | |
| 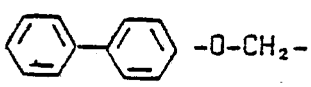 | 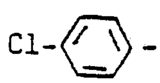 | |
| 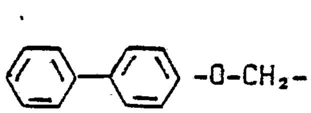 | 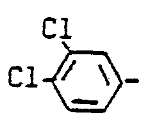 | |
| 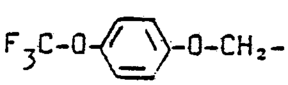 | 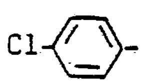 | |

| $R^1$ | $R^2$ | Physikal.Konstante |
|---|---|---|
| 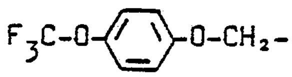 | 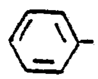 | |
| 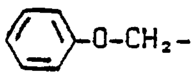 | 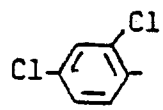 | |
| 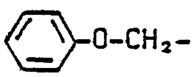 | 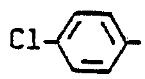 | |
| 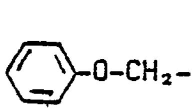 | 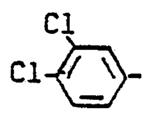 | |
| 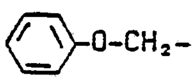 | 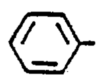 | |
| 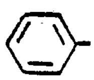 | 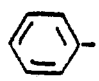 | $n_D^{20}$: 1,594 |
| 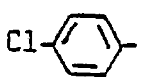 | 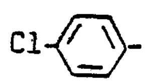 | $n_D^{20}$: 1,600 |
| 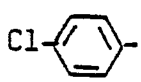 | 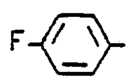 | $n_D^{20}$: 1,576 |
| 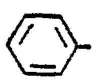 | 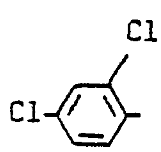 | Fp. 80°C |
| 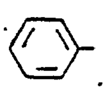 | 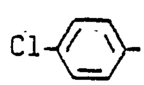 | Fp. 59°C |

| $R^1$ | $R^2$ | Physikal.Konstante |
|---|---|---|
| 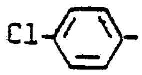 | 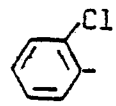 | $n_D^{20}$: 1,6059 |
| 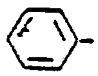 | 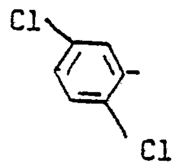 | $n_D^{20}$: 1,6048 |
| 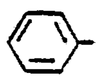 | 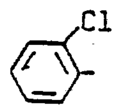 | $n_D^{20}$: 1,6019 |
| 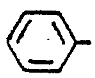 | 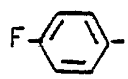 | $n_D^{20}$: 1,574 |
| 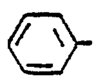 | 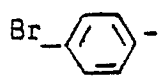 | Fp. 77-78°C |
| 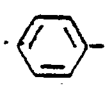 | 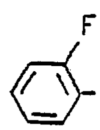 | $n_D^{20}$: 1,5763 |
| 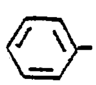 | 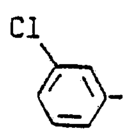 | $n_D^{20}$: 1,5943 |
| 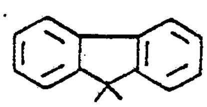 | | Fp. 101°C |

| $R^1$ | $R^2$ | Physikal.Konstante |
|---|---|---|
| 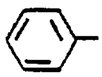 | $CH_3$- | $n_D^{2o}$: 1,54o3 |
| 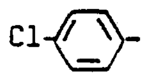 | $CH_3$- | $n_D^{2o}$: 1,5528 |
| 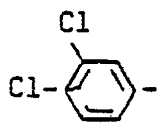 | $CH_3$- | $n_D^{2o}$: 1,5763 |
| 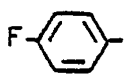 | $CH_3$- | Kp. 78°C/o,2 mm Hg |
| 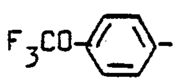 | $CH_3$- | $n_D^{2o}$: 1,476 |
| 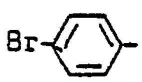 | $CH_3$- | $n_D^{2o}$: 1,564 |
| 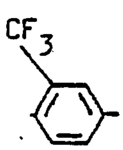 | $CH_3$ | $n_D^{2o}$: 1,4749 |
| 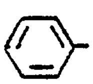 | $C_3H_7$ -iso | $n_D^{2o}$: 1,529o |
| 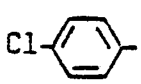 | $C_3H_7$-iso | $n_D^{2o}$: 1,54o5 |
| 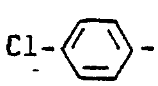 | $C_2H_5$- | $n_D^{2o}$: 1,5431 |

| $R^1$ | $R^2$ | Physikal. Konstante |
|---|---|---|
| 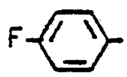 | $C_3H_7$-iso | $n_D^{20}$: 1,5125 |
| 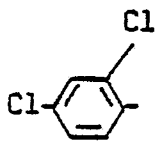 | $C_2H_5$ | $n_D^{20}$: 1,5511 |
| 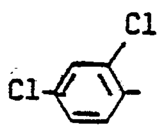 | $CH_3$- | $n_D^{20}$: 1,5615 |
| 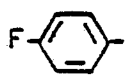 | 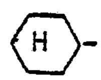 | $n_D^{20}$: 1,5342 |
| 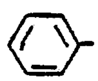 | 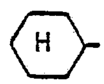 | $n_D^{20}$: 1,5487 |
| 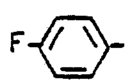 | $C_2H_5$ | $n_D^{20}$: 1,5138 |
| 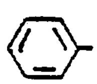 | $C_2H_5$ | $n_D^{20}$: 1,5362 |
| 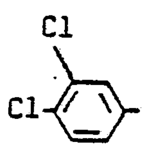 | $C_2H_5$ | $n_D^{20}$: 1,5612 |

Verwendungsbeispiele

Die nachfolgenden Verbindungen werden als Vergleichssubstanzen eingesetzt:

(A) 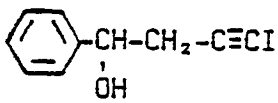

1-Jod-4-phenyl-1-butin-4-ol

(B) 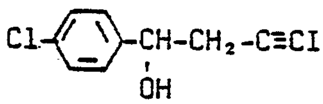

4-(4-Chlorphenyl)-1-jod- 1-butin-4-ol

Beispiel A

Erysiphe-Test (Gerste) / protektiv

Lösungsmittel: 100 Gew.-Teile Dimethylformamid
Emulgator: 0,25 Gew.-Teile Alkyl-aryl-polyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 3 und 13.

Beispiel B

Puccinia-Test (Weizen) / protektiv

Lösungsmittel: 100 Gew.-Teile Dimethylformamid
Emulgator: 0,25 Gew.-Teile Alkyl-aryl-polyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 8, 9, 12, 19, 22 und 32.

0066761

Beispiel C

Phytophthora-Test (Tomate) / protektiv

Lösungsmittel: 4,7 Gew.-Teile Aceton
Emulgator: 0,3 Gew.-Teile Alkyl-aryl-polyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20°C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 3, 5, 8, 13 und 14.

Patentansprüche

1) Substituierte 1-Jod-1-butin-4-ole der allgemeinen Formel (I),

$$I-C\equiv C-CH_2-\overset{R^1}{\underset{R^2}{C}}-OH \qquad (I)$$

in welcher

$R^1$ für Alkyl, gegebenenfalls substituiertes Aryl, Aralkyl oder Aryloxyalkyl steht,

$R^2$ für Alkyl mit mehr als einem C-Atom, Halogenalkyl, gegebenenfalls substituiertes Cycloalkyl oder Aryl steht und für den Fall, daß $R^1$ für gegebenenfalls substituiertes Aryl, Aralkyl oder Aryloxyalkyl steht, außerdem für Methyl steht oder

$R^1$ und $R^2$ gemeinsam mit dem C-Atom an das sie gebunden sind für Fluoren-9-yl stehen.

2) Substituierte 1-Jod-1-butin-4-ole der allgemeinen Formel (I), in der

$R^1$ für gegebenenfalls substituiertes Aryl mit 6 bis 10 C-Atomen, Aralkyl und Aryloxyalkyl mit 6 bis 10 C-Atomen im Arylteil und 1 bis 4 C-Atomen im Alkylteil steht,

$R^2$ für $C_1$-$C_{10}$-Alkyl, $C_1$-$C_{10}$-Halogenalkyl mit 1 bis 13 Halogenatomen, gegebenenfalls substituiertes Cycloalkyl mit 3 bis 7 C-Atomen, oder Aryl mit 6 bis 10 C-Atomen steht oder

$R^1$ und $R^2$ gemeinsam mit dem C-Atom an das sie gebunden sind für Fluoren-9-yl stehen.

3) Substituierte 1-Jod-1-Butin-4-ole der allgemeinen Formel (I), in der

$R^1$ für gegebenenfalls substituiertes Phenyl, Benzyl oder Phenyloxymethyl steht,

$R^2$ für $C_1$-$C_4$-Alkyl wie Methyl, Ethyl, Propyl, iso-Propyl, Butyl, sek.-Butyl, tert.-Butyl, $C_1$-$C_4$-Halogenalkyl mit 1 bis 3 Halogenatomen, Cyclohexyl oder gegebenenfalls substituiertes Phenyl steht oder

$R^1$ und $R^2$ gemeinsam mit dem C-Atom an das sie gebunden sind für Fluoren-9-yl stehen.

4) Verfahren zur Herstellung von substituierten 1-Jod-1-butin-4-olen der allgemeinen Formel (I),

$$I-C\equiv C-CH_2-\underset{R^2}{\overset{R^1}{C}}-OH \qquad (I)$$

in welcher

$R^1$ für Alkyl, gegebenenfalls substituiertes Aryl, Aralkyl oder Aryloxyalkyl steht,

$R^2$ für Alkyl mit mehr als einem C-Atom, Halogenalkyl, gegebenenfalls substituiertes Cycloalkyl oder Aryl steht und für den Fall, daß $R^1$ für gegebenenfalls substituiertes Aryl, Aralkyl oder Aryloxyalkyl steht, außerdem für Methyl steht oder

$R^1$ und $R^2$ gemeinsam mit dem C-Atom an das sie gebunden sind für Fluoren-9-yl stehen,

dadurch gekennzeichnet, daß man 1-Butin-4-ole der Formel (II),

$$H-C\equiv C-CH_2-\underset{R^2}{\overset{R^1}{\underset{|}{\overset{|}{C}}}}-OH \qquad (II)$$

in welcher

$R^1$ und $R^2$ die oben angegebenen Bedeutungen haben,

mit Jod in Gegenwart einer basischen Verbindung und gegebenenfalls in Gegenwart eines Verdünnungsmittels umsetzt.

5) Verfahren nach Anspruch 4, dadurch gekennzeichnet, daß in einem Verdünnungsmittel umgesetzt wird.

6) Verfahren nach Anspruch 4, dadurch gekennzeichnet, daß bei einer Temperatur von 0 bis 50°C umgesetzt wird.

7) Pflanzenschutzmittel, dadurch gekennzeichnet, daß sie mindestens ein 1-Jod-1-butin-4-ol der Formel (I) gemäß Anspruch 1 enthalten.

8) Verwendung von 1-Jod-1-butin-4-olen der Formel (I) gemäß Anspruch 1 zur Bekämpfung von Pflanzenschädlingen.

9) Verfahren zur Bekämpfung von Pflanzenschädlingen, dadurch gekennzeichnet, daß man 1-Jod-1-butin-4-ole der Formel (I) gemäß Anspruch 1 auf pflanzenpathogene Schädlinge und/oder ihren Lebensraum einwirken läßt.

10) Verfahren zur Herstellung von Pflanzenschutzmitteln, dadurch gekennzeichnet, daß man 1-Jod-1-butin-4-ole der Formel (I) gemäß Anspruch 1 mit Streckmitteln und/oder oberflächenaktiven Mitteln vermischt.

Europäisches Patentamt

## EUROPÄISCHER RECHERCHENBERICHT

0066761

Nummer der Anmeldung

EP 82 10 4486

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| --- | --- | --- | --- |
| D,X | FR-A-1 404 792 (MEIJI SEIKA KAISHA)<br>*Zusammenfassung* & DE - A - 1 217 368<br>--- | 1-10 | C 07 C 33/50<br>C 07 C 33/48<br>C 07 C 33/42<br>C 07 C 33/44<br>C 07 C 35/52<br>C 07 C 43/32<br>A 01 N 31/04 //<br>C 07 C 29/62 |
| X | CHEMICAL ABSTRACTS SERVICE REGISTRY HANDBOOK NUMBER SECTION, Band R7, 1965-1971, Registry Numbers 30300-01-7 through 33913-68-7, Seite 11487R, Columbus Ohio (USA); "30876-54-1 Benzenemethanol, alpha-(3-iodo-2-propynyl)-alpha-methyl- C11H11IO". *Seite 11487R, Spalte 2, Zeilen 21,22; R.N.30876-54-1*<br>--- | 1,2,3 | |
| A | CHEMICAL ABSTRACTS, Band 85, Nr. 21, 22. November 1976, Seite 492, Nr. 159417t, Columbus Ohio (USA);<br>& JP - A - 76 98 205 (ONO PHARMACEUTICAL CO., LTD.) (30-08-1976)<br>*Insgesamt*<br>----- | 1 | RECHERCHIERTE SACHGEBIETE (Int. Cl. 3)<br>C 07 C 33/00<br>C 07 C 35/00<br>A 01 N 31/00<br>C 07 C 43/00 |

Der vorliegende Recherchenbericht wurde für alle Patentansprüche erstellt.

| Recherchenort | Abschlußdatum der Recherche | Prüfer |
| --- | --- | --- |
| DEN HAAG | 20-08-1982 | DELHOMME H.A. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument